# EUROPEAN PATENT APPLICATION

(11) **EP 4 796 927 A1**
(43) Date of publication of application: **26.08.2026**
(21) Application number: 25159090.7
(22) Date of filing: 20.02.2025
(51) Int. Cl.: G01N 33/02

(54) **ANIMAL FOODSTUFF MONITORING DEVICE AND SYSTEM**

(71) Applicant: Quanturi Oy, 00260 Helsinki (FI)
(72) Inventor: Pesonen, Nadine, 00350 Helsinki (FI); Raju, Geet, 00560 Helsinki (FI); Huyghe, Marie, 00370 Helsinki (FI); Veijonen, Teppo, 00100 Helsinki (FI)
(74) Representative: Kolster Oy Ab

(57) **Abstract**

The present disclosure relates to a method of monitoring an animal foodstuff (54) comprising measuring one or more property of the foodstuff using a sensor (22) on a monitoring device (2) inserted into the foodstuff (54) and storing said measurement data on the monitoring device (2). The monitoring device (2) receives data (38) relating to one or more processing stage (36) of the foodstuff.

## Description

The present disclosure relates to a monitoring device for monitoring animal foodstuff, and method and system of using said device.

### Background of the Invention

Vegetable animal foodstuff, known as fodder, is typically harvested, transported to a further location and then stored for a period of time before usage. During storage or transportation etc., the quality of fodder may decrease due to a number of variables. For example, a 10% loss in fodder digestibility can cause a decrease of 2kg of potential daily milk production. This equals a loss of around 315$/cow/year according to the milk prices from December 2024. Moreover, the exact / precise quality of the fodder is difficult to determine, even for an experienced farmer.

At present, there is no automated means to track the quality and/or other processing parameters of the foodstuff through its lifecycle from harvesting all the way to feeding of the animals. This makes it difficult to determine the composition of ration required to maintain sufficient production.

The present invention aims to overcome or ameliorate one or more of the above problems.

### Statement of Invention

According to a first aspect, there is provided: a method of monitoring an animal foodstuff comprising: measuring one or more property of the foodstuff using one or more sensor on a monitoring device inserted into the foodstuff and storing said measurement data on the monitoring device; and receiving by the monitoring device, data relating to one or more processing stage of the foodstuff.

The monitoring device may comprise a plurality of sensors. The sensor may measure a plurality of properties of the foodstuff (e.g. to provide a multifunctional sensor). The sensor may measure a single property of the foodstuff (e.g. multiple sensors measure respective properties). The plurality of sensors may measure the respective properties simultaneously. The monitoring device may provide a probe.

The method may comprise storing said processing stage data on the monitoring device. The processing stage data may be stored in volatile and/or non-volatile memory.

The method may comprise transmitting the measurement data and/or the processing stage data to a remote device. The measurement data and/or the processing stage data may be transmitted during/between any processing stage and/or when all processing stages have been completed. The measurement data and/or the processing stage data may be stored on a remote server (e.g. stored in the cloud).

The property of the foodstuff may comprise one or more of: temperature; moisture; relative humidity; pressure; or pH of the foodstuff. The monitoring device may comprise respective sensors to measure said properties. The property of the foodstuff may comprise a nutritional property.

The processing stage may comprise one or more of: production; transportation; storage or usage of the foodstuff. The production stage may comprise a harvesting stage. The production stage may comprise a packaging or baling stage.

The processing stage data may comprise one or more of: a geographical locating of the processing stage; weather conditions during processing; an initial nutritional content of the foodstuff; an identifier of a vehicle; a storage time or type (e.g. in a container, silo, enclosed or partially enclosed); or a feed ration of the foodstuff. The processing stage data may comprise an identifier of the fodder or unit/batch thereof. The identifier may be unique to each unit/batch. The identifier may be the same and/or associated with an identifier on the monitoring device. The processing stage data may comprise one or more of: a crop variety/species; the amount of crop; a nutritional content of the crop (e.g. at the production stage); a farmer/producer identifier; an identifier of the fodder and/or batch/unit thereof (e.g. a bale or container); and location of foodstuff at the start/end of transportation.

The monitoring device may comprise a sensor configured to detect an initiation event. The monitoring device may initiate measuring of one or more property of the foodstuff in response to said initiation event. The sensor may be configured to detect insertion of the monitoring device into the foodstuff and/or release from an applicator.

The monitoring device may comprise a sensor configured to detect a termination event. The monitoring device may terminate measuring of one or more property of the foodstuff in response to said termination event. The sensor may be configured to detect removal of the monitoring device into the foodstuff

The monitoring device may be configured to pair with a remote device during the processing stage. The monitoring device may receive and transmit said processing stage data from and to the remote device. The monitoring device may pair wirelessly with the remote device.

The method may comprise receiving processing stage data at each processing stage. The method may comprise storing the data for each processing stage on the monitoring device. The method may comprise concurrently continuously storing measurement data on the monitoring device. The method may comprise transmitting all the data for each processing stage and the measurement data after the processing stages are complete.

Measurement data may be recorded at least every 6 hours, preferably, at least every 3 hours. Measurement data may be recorded at least every hour.

According to a further aspect, there is provided: a monitoring device configured to monitor an animal foodstuff comprising: one or more sensor on the monitoring device configured to be inserted into the foodstuff and to measure one or more property of the foodstuff; a controller configured to store said measurement data on the monitoring device; and the controller configured to receive data relating to one or more processing stage of the foodstuff.

Said processing stage data may be stored on the monitoring device.

The controller may comprise a processor and/or memory. The monitoring device may comprise a communication interface. The communication interface may comprise a wireless interface.

The monitoring device may comprise a first end configured to be received in the foodstuff. The monitoring device may comprise a second end configured to protrude from the foodstuff. The second end may be wider than the first end (e.g. to provide a collar or rim). The measurement sensors may be provided near or at the first end. The second end may comprise one or more identifier. The identifier may comprise one or more: a visual indicia (e.g. human or machine-readable code); a machine interrogable identifier (e.g. RFID).

The monitoring device may comprise a sensor configured to detect removal and/or insertion of the monitoring device into the foodstuff. The sensor may comprise: a magnetic sensor; or release switch.

According to a further aspect, there is provided: a monitoring system configured to monitor an animal foodstuff comprising: a monitoring device comprising one or more sensor configured to be inserted into the foodstuff and to measure one or more property of the foodstuff; a controller on the monitoring device configured to store said measurement data on the monitoring device; and a processing stage device provided at one or more processing stage of the foodstuff.

The controller may be configured to receive data relating to one or more processing stage of the foodstuff from the remote device. Said processing stage data may be stored on the monitoring device. The processing stage device may transmit the identifier and the respective processing stage data to a remote server and/or to the monitoring device.

According to a further aspect, there is provided: a method of monitoring an animal foodstuff comprising measuring one or more property of the foodstuff using one or more sensor on a monitoring device inserted into the foodstuff, the monitoring device comprising one or more identifier; determining via a device at a processing stage the identifier on the monitoring device and associating the identifier with data relating to the processing stage.

The processing stage device may collect/collate the processing stage data at the respective processing stage. The processing stage device may store the data locally. The processing stage device may transmit data to a remote device. The remote device may comprise a remote server and/or cloud storage device. The remote device may collate/aggregate from each processing stage. The remote device may receive measurement data from the monitoring device. The processing stage device may pair/connect to the monitoring device. The monitoring device may transmit measurement data to the remote device. The remote device may transmit measurement data and/or processing stage data to a further device (e.g. a user device).

The processing stage device may be configured to read/extract the identifier on the monitoring device. The processing stage device may comprise a camera, optical device, or interrogating device to read/interrogate said identifier.

According to a further aspect, there is provided: a monitoring system configured to monitor an animal foodstuff comprising: a monitoring device comprising one or more sensor configured to be inserted into the foodstuff and to measure one or more property of the foodstuff and comprising one or more identifier; a device provided at one or more processing stage of the foodstuff, and comprising a means to determine the identifier on the monitoring device; and where the processing stage device comprises a controller configured to associate the identifier with data relating to the processing stage.

Measurement data and processing stage data may be stored on both the monitoring device and the remote device.

According to a further aspect, there is provided: an applicator for inserting a monitoring device into a foodstuff comprising: a magazine configured to receive one or more monitoring device, the magazine comprising at least one outlet; and a pushing mechanism configured to push at least one of the monitoring devices out from the magazine via the outlet.

The magazine may comprise a tapered/conical shape. The magazine may be rotatable/movable to move a monitoring device into alignment with the pusher. The pusher comprises a sufficient stroke length to push the monitoring device out from the magazine. The pusher may comprise an actuation mechanism to extend/retract the pusher. The pusher may be connected to the actuation mechanism via a rack and pinion.

The magazine may comprise a trigger for triggering the sensor to indicate insertion of the monitoring device into the food stuff.

The applicator may comprise a perforator configured to provide a hole in the foodstuff such that the monitoring device may be inserted thereinto. The perforator may comprise a needle, drill or spike. The perforator may extend through the magazine.

The applicator may comprise a sensor system to ensure alignment with the hole made by the perforator. The sensor system may ensure alignment of an outlet of the magazine with the hole.

The applicator may be fixed to a baler, harvester or other agricultural processing apparatus.

According to a further aspect, there is provided: an extractor for removing a monitoring device from a foodstuff comprising: a jaw configured to grip an end of the monitoring device, the jaw comprising two movable jaw portions.

The jaw may comprise a cavity to receive a portion of the monitoring probe.

Any aspect of the invention may be combined with any other aspect of the invention where practicable.

### Description

Embodiments of the present invention are described below, by way of example only, with reference to the accompanying drawings:
**Figure 1** shows a schematic side view of a monitoring device;
**Figure 2** shows a schematic end view of the monitoring device;
**Figure 3** shows a schematic view of a monitoring system;
**Figure 4A** shows a schematic view of a first monitoring process;
**Figure 4AB** shows a schematic view of a second monitoring process;
**Figure 5** shows a schematic side view of a probe applicator magazine;
**Figure 6** shows a schematic side view of a probe applicator perforator;
**Figure 7** shows a schematic side view of a probe applicator;
**Figure 8** shows a schematic side view of a probe extractor.

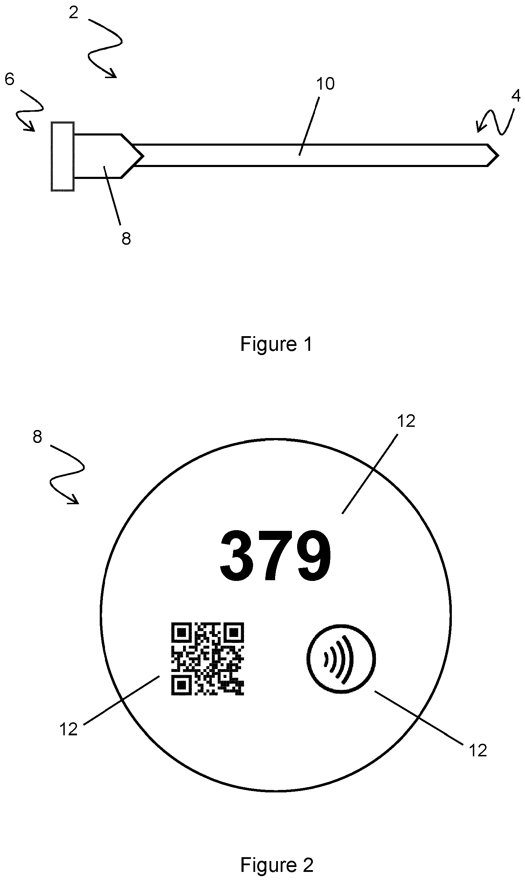

A monitoring device 2 is shown in figures 1 and 2. The monitoring device 2 is configured to be inserted into an animal foodstuff or fodder. The foodstuff may comprise a grass and/or legume. The foodstuff may be grown in a field containing both a grass and legume, or a field comprising only a grass or a legume. The foodstuff may comprise one or more of, inter alia: ryegrass; timothy; brome; fescue; Bermuda grass; orchard grass; alfalfa (Lucerne); clover (red, white and/or subterranean); cereals (e.g. grains); any other herbaceous plant or, grass or legume; and/or combinations thereof.

Typically, the foodstuff is fully or partially cut and harvested in a conventional manner. Further processing to the foodstuff may be provided, for example, one or more of: raking; tedding; baling; chopping; drying; dehydration; compaction; and wrapping. The processed foodstuff may provide one or more of: hay; silage; haylage; straw; sprouted grains; legumes; oils; or pellets of said crops. The harvested and/or processed foodstuff provides the fodder accordingly.

The fodder may be transported to one or more further location. Transportation may comprise gathering of the fodder with a loader, forklift, or motorized equipment to be taken directly or by means of trailers, containers for transportation to storage. Transportation might include a single or multiple legs by air, road, or sea until final destination for storage and usage.

The fodder may be stored for later use. Storage of the fodder may comprise: storage in warehouses, bunkers, silos etc; open air; under a tarpaulin or cover; or in wrapping (e.g. in silage wrap). Fodder may be stored in countable/discrete units (e.g. bales or the like) or stacked in bulk.

The monitoring device 2 comprises a first end 4 configured to be inserted into the fodder. The first end 4 may be pointed or tapered to allow easy insertion. A second end 6 comprising a housing 8. The housing 8 may contain electronics or the like, as will be described later. A shaft 10 extends between the first end 4 and the second end 6. The length of the shaft 10 may be adapted according to needs of the user. Typically, the shaft is between 10cm and 100cm. The second end 6 is wider than the shaft 10 (e.g. to provide a collar, sleeve or rim). This helps to prevent over-insertion of the device.

The device 2 comprises a plastic or polymeric casing. In some embodiments, the monitoring device 2 may comprise a biodegradable or digestible (e.g. edible by animals) material. For example, the monitoring device 2 may be manufactured from straw or other food product. The food product may be compressed/agglomerated to form a rigid material. Any electronic components on the monitoring device 2 may be manufactured using polymer material or other edible/biodegradable materials.

As shown in figure 2, the second end 6 comprises one or more identifier 12. The identifier 12 is configured to identify the device 2 and the fodder accordingly. The identifier 12 may comprise a visual indicia, for example, one or more of: written indicia (e.g. numbering, writing, alphanumeric code, or other code etc.); and/or machine-readable code (e.g. QR code or barcode). The identifier 12 may comprise a machine interrogable identifier, for example, an RFID device. The identifier may comprise a passive device (e.g. powered by an interrogation signal). The identifier may comprise an active device. For example, the active device may be configured to broadcast an identifying signal and/or establish a connection to an interrogating device. The active device may be configured to connect to an interrogating device via any suitable protocol, for example, one or more: Bluetooth; Wi-Fi; ZigBee; LoRa; NbloT; GSM; 2G; 3G; 4G; 5G; NFC; IrDA etc. The identifier 12 may be provided on or in the housing 8 according to the exact configuration.

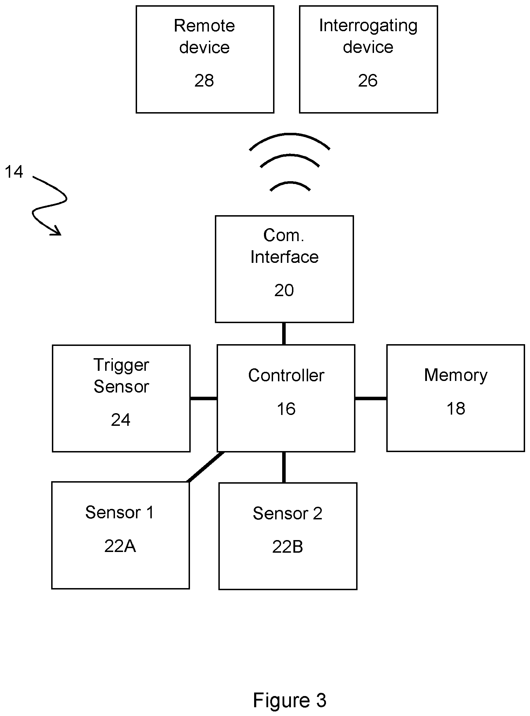

The monitoring device 2 forms part of a monitoring system 14 shown schematically in figure 3. The monitoring system 14 is configured to monitor one or more property of the fodder or the processing characteristics thereof. Data is recorded in the device, which may then be retrieved at a later time. The device may provide a datalogger. The device 2 therefore acts as monitoring device, as well as identifying the fodder.

The monitoring system 14 comprises a controller 16. The controller 16 may comprise a processor. The processor may comprise any suitable for, for example: logic components; standard integrated circuits; application-specific integrated circuits (ASIC); system-on-a-chip (SoC); application-specific standard products (ASSP); microprocessors; microcontrollers; digital signal processors; special-purpose computer chips; field-programmable gate arrays (FPGA); and other suitable electronics structures. The monitoring system 14 may comprise memory 18. The memory may include volatile memory (e.g. RAM) and/or non-volatile memory. The apparatus may comprise a communication interface 20. The communication interface may comprise a wired or wireless interface (e.g. Wi-Fi; mobile/cellular interface; Bluetooth; NFC; local or near field interface, HF/UHF communication). It can be appreciated that the exact form of the hardware and/or software used to implement the present system is not pertinent to the invention at hand.

The identifier 12 may be incorporated into the monitoring system. For example, the monitoring device 2 may be identified via the communication interface 20. The controller may send an identification signal or the like. Thus, the monitoring system 14 provides both monitoring and identification. In other embodiments, the identifier 12 and the monitoring system 14 operate independently.

The monitoring device 2 may comprise one or more sensor 22. The sensor(s) 22 are configured to measure one or more property of the fodder, for example, one of more of: temperature; moisture; relative humidity; pressure; or pH. Appropriate sensor types are provided according to the desired measurements. Although two such sensors 22A, B are shown in figure 3, it can be appreciated that any amount may be provided. The sensors 22 may be configured to detect one or more of the above properties. The sensors 22 may be provided at or near the first end 4 of the monitoring device 2. The monitoring system 14 is typically provided at the second end 6 of the device. Wires or the like may extend between the sensors 22 and monitoring system 14 through the shaft 10.

The sensors 22 are operatively connected to the monitoring system 14 (e.g. the controller 16). Data recorded by the sensors 22 is stored in the monitoring system 14. The data is stored in the memory 18. The sensors 22 are configured to periodically measure the properties of the fodder. For example, the sensors 22 may measure the properties of the fodder at least every 12 hours, at least every 6 hours, or at least every 3 hours. Typically, the sensors 22 may measure the properties of the fodder at least once an hour. This allows collection of high-quality data representative of real-world conditions of the fodder.

The sensors 22 continually monitor properties of the fodder whilst the device is inserted into the fodder. The device 2 comprises a trigger sensor 24 to detect when the monitoring device 2 is inserted into the fodder. When insertion of the monitoring device 2 is detected, the monitoring system 14 is configured to start data collection from the sensors 22. When removal of the monitoring device 2 is detected, the monitoring system 14 is configured to end data collection from the sensors 22. This allows automatic starting/ending of data collection.

In some embodiments, the monitoring system 14 comprises a passive device. The monitoring system 14 is therefore not powered. Power is provided by an interrogating device 26. The sensors 22 may comprise their own power supply, the sensors 22 passively provide data to the controller 16/memory 18. In some embodiments, the entire system comprises a passive device and is powered from a remote or interrogating device. For example, a device may be provided in the same location as the fodder and the device is configured to periodically power/interrogate the monitoring system 14. In other embodiments, the monitoring system 14 comprises an active device. The monitoring system 14 comprises a power supply accordingly (e.g. a rechargeable battery). In some embodiments, the monitoring system 14 comprise an active-passive hybrid system. For example, the monitoring system 14 is configured to measure and process sensor data, however, the memory 18 may be passively written/read by the interrogating device 26 to save power.

In some embodiments, the monitoring system 14 may be manually activated. For example, the device 2 may comprise a switch, button or other manual input to initiate data collection. In some embodiments, the monitoring system 14 may be activated electronically/remotely. For example, a remote device may transmit a signal to initiate/end data collection.

The monitoring system 14 may be configured to transmit sensor data and/or other data. The data may be transmitted to a remote device 28. The remote device may comprise, for example, any of: a mobile/cellular device; a tablet device; laptop device; desktop device; server; cloud server etc. This may allow real-time observation of the measurements by a user.

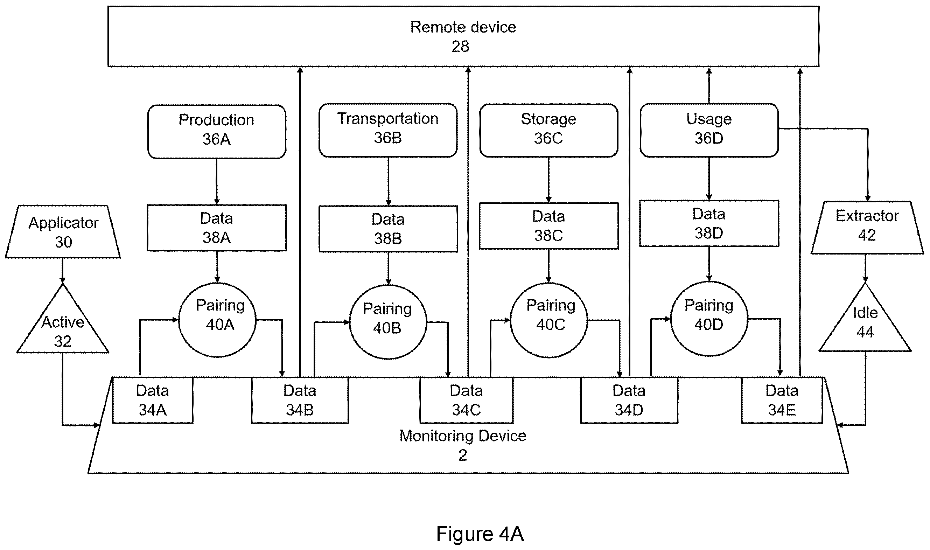

Use of the monitoring system 14 is described with reference to figure 4A. The monitoring system 14 is used to record data at each processing stage thereof. At each processing stage, the monitoring system 14 is connected with a remote device, and data regarding the processing stage is transmitted to the monitoring system 14. The data is recorded within the memory 18 on the device 2. The data for each processing stage is therefore recorded on a single device, which can then be extracted at the final stage of use, for example, at the feeding stage. The measurement data may also be extracted at this stage, for example, to determine the quality of the fodder.

An applicator 30 is used to insert the monitoring device 2 into the fodder. The applicator 30 will be described in detail later. During insertion, the trigger sensor 24 is activated. This sets an "active" mode 32 in the monitoring system 14, and the sensors 22 begin collecting data. The sensor data 34A is stored on the device 2. Sensor data 34A is collected continuously throughout the process. The active mode 32 may also initiate connections with other device (e.g. remote device 28) and/or perform any other functions, as required.

In a first processing step 36A, the fodder is produced. During this process, fodder may be produced by means of baler, wrapper, combine and/or chopper. The processing gives the raw crop (e.g. grass and legumes) its final form (e.g. bale, pellets or loose material). The processing may provide a packaging or baling stage. Data 38A regarding the production of the fodder is collected. The data 38A may be stored in memory on a device provided at the processing stage. The production data 38A may comprise any of the following:
- A geographical location of the crop. The location may comprise coordinates (e.g. latitude and longitude) of the crop. The location may define a zone, boundary or area of the crop. The location may be granular (e.g. limited to discrete values of latitude and longitude or regions of a country etc.) or may be high accuracy (e.g. down to 1m or 10m accuracy). The location may be a simple descriptor (e.g. "Field 1", "Field 2"). Location data may be obtained via any suitable means, inter alia: satellite/aerial imagery; cellular networks; localised geo-positioning sensors (e.g. GPS sensors); digital mapping services; authority (e.g. government or government agency) registries; or manual input. Location data may be collected for example, via a user's mobile (cellular) phone or tracking systems in agricultural machinery.
- Crop coverage. This is the amount of area the crop covers relative to the geographic area the crop is contained. This may be provided as a percentage or fraction of the total area. The crop coverage may be determined in a similar manner to the geographical area. The crop coverage may be determined from the yield collected from a specific area (e.g. the yield determined during harvest thereof via an agricultural machine).
- The plant species or variety of the crop. The species/variety may be obtained one or more of: authority (e.g. government or government agency) registries; national or producer databases; laboratory analysis; or manual input (e.g. when the user simply knows the species/variety).
- The cutting time or timeline of the crop (i.e. at what time(s) the crop was cut). The cutting time/timeline may comprise date and/or time data. For example, the cutting time/timeline may comprise [day,month,year] data. The data may comprise a series of date/time data points, where cutting is performed multiple times. Cutting data may be obtained from one or more: harvesting/cutting equipment having tracking or monitoring capabilities; local registries or databases; or manual input.
- The cutting frequency of the crop. The frequency may be determined over a specific time period (e.g. week, month, year etc.). Typically, the cutting frequency is the number of times the grass is cut in a calendar year or growing season. Cutting data may be obtained from one or more: harvesting/cutting equipment having tracking or monitoring capabilities; local registries or databases; or manual input.
- The processing applied to the crop/fodder. This may comprise the type of processing: for example: raking; tedding; baling; chopping; drying; dehydration; compaction; and wrapping. The processing parameter may further comprise one or more of: the (time) length of a given processing step; a drying rate; a moisture profile (e.g. starting and/or finishing moisture level of the crop); start and/or finish density of the fodder; a wrapping material. Processing data may be obtained from one or more: harvesting/cutting equipment having tracking or monitoring capabilities; local registries or databases; or manual input.
- The amount of fodder produced. For example, the weight, volume, density of the fodder.
- The farming technique or other agricultural processing of the crop. The farming technique may indicate practices relating one or more of: seeding (e.g. seeding date, seeding coverage, seeding density); fertilisation (e.g. type, amount, coverage thereof); or soil manipulation (e.g. amount or type of pH raising/lowering agents). Data may be obtained from one or more: harvesting/cutting equipment having tracking or monitoring capabilities; local registries or databases; or manual input.
- The soil composition in which the crop is grown. The soil composition may comprise one or more: organic content; mineral content; or water content. Data may be obtained from, one or more of: authority (e.g. government or government agency) registries; national databases; laboratory analysis; or manual input.
- The condition of the fodder after processing. The data may comprise one or more of: temperature; moisture level; pH; form factor; or density of the fodder. Measurements may be for a single instance or may be made over a period of time (i.e. multiple time spaced measurements are made). Data may be obtained from one or more of: satellite imagery; aerial photography (e.g. via drones); harvesting/cutting equipment having tracking or monitoring capabilities; local registries or databases; local analysis (e.g. with portable sensors); laboratory analysis; or manual input.
- Identifier of the farmer/producer. For example, a unique identifier, name, address may be provided.
- Identifier of the fodder. This can be for a single fodder or a batch/unit of fodder. For example, the identifier may be for a single bale or container or fodder. A single identifier may be provided for multiple unit/batches, for example, where such unit/batches remain together and/or in the same environmental conditions. The identifier may comprise the identifier 12 and/or be associated with said identifier 12 on the monitoring device 2. Thus, the monitoring device 2 and the foodstuff are associated with one another.
- Weather conditions. The weather conditions may be indicative of the weather conditions during growing of the crop, harvesting of the crop; processing of the crop; after processing of the crop; and/or during storage of the fodder. Data may be obtained across one or more time period of the crop growing/processing/storage stage and/or may be continuously recorded. The weather conditions may comprise one or more of: temperature; precipitation amount (e.g. rainfall); humidity; wind direction; wind speed; atmospheric pressure; growing degree days; or dew points. Weather data may be obtained from one or more of: satellite imaging or data; local or national weather databases; proprietary databases; local registries; local sensor measurements; or human inputs.
- Fodder nutritional composition:
   - Protein content. For example, crude proteins (CP) and/or specific amino acid levels.
   - Carbohydrate content. For example, non-fibre carbohydrates (NFC); water-soluble carbohydrates (WSC); ether-soluble carbohydrates (ESC); and/or specific sugar contents (e.g. fructose, sucrose, fructan).
   - Fat content.
   - Fibre content. For example, neutral detergent fibres (aNDF); acid detergent fibres (ADF); and/or acid detergent lignin (ADL).
   - Other broad indicators of nutritional value. For example, total digestible nutrients (TDN); relative feed value (RFV), relative feed quality (RFQ); and/or calorific content.
   - Moisture content and/ or dry content. For example, the volume/weight percentage moisture.
   - Mineral/vitamin content. For example, one or more of: Calcium (Ca); Phosphorus (P); Sodium (Na); Chloride (Cl); Magnesium (Mg); Potassium (K); Sulphur (S) Cobalt (Co); Copper (Cu); Fluoride (F); Iron (Fe); Manganese (Mn); Molybdenum (Mo); Selenium (Se); or Zinc (Zn)

The data 38A may originate from one or more sources. For example, data 38A may be manually input or collected automatically. Data sources may include agricultural production and/or processing apparatus (e.g. harvesters, bailers, tractors etc.). The data may be stored on a processing apparatus, for example, a baler. The data may be manually collected/collated (e.g. using the remote device 28). Data 38A may be stored locally. Data 38A may be stored on server or cloud storage (i.e. remotely). A device is provided at the processing stage 36A, which retrieves said data 38A and transmits the data 38A to the monitoring device 2. Data 38A may relates to multiple batches of foodstuff (e.g. where they comprise the same values).

The monitoring device 2 pairs with the production data 38A source (e.g. on the processing apparatus or the remote device). The monitoring device 2 pairs with the production data 38A source using a pairing process 40A. Such a pairing process may use any suitable technique, depending on the specific protocol used. In some embodiments, the monitoring device 2 may connect to the data source using a wired connection (e.g. ISOBUS). Transmission of the data 38A may be automatically initiated (e.g. during a baling process) or may be user initiated.

The production data 38A is stored on the monitoring device 2. This is combined with the measurement data 34A already on the monitoring device 2 to create an updated dataset 34B. Any or all of the data 34B may be transmitted to the remote device 28, for example, to cloud storage. This may help to ensure data integrity, for example, in case of a device failure. Data may be stored on removable storage (e.g. SD cards or the like). Data may be accessible via removable storage, for example, a removable USB drive or writable CD/DVD. The data may be transmitted to the remote device 28 at any time in the process. For example, the user may wish to inspect the data after each processing stage 36.

The process is repeated for the next processing stage, transportation 36B. Transportation of the fodder may be by air, sea, train and/or or road. The fodder may be transported by trailers or containers. The fodder may be loaded into containers/trailers by loaders, forklifts or any picking or lifting equipment. Transportation can be from the harvest area (e.g. a farm) to a distant storage or with the harvest area itself (e.g. to separate location on the farm). Similarly, transportation data 38B is transmitted to the monitoring device 2 via a pairing process 40B. The transportation data 38B may comprise any of the following:
- Identifiers of the fodder and/or the transportation equipment used. For example, a registration number or other unique identifier of the trailer or vehicle used or fodder.
- The type of vehicle used. For example, road vehicle, water vessel or air vessel.
- Fodder weight, volume or density.
- Location of the fodder at the start, end or during transportation. For example, coordinates of the path taken by the fodder. The device 2 may comprise a position system (e.g. GPS) to record location data. Location data may be manually input at the start/end of the transportation. Location data may be inferred from a starting/ending location of the fodder. For example, the location can be inferred from the start location of a farm and an end location of a port.
- Physical conditions of the fodder. For example, temperature, moisture level, or pH.
- Other transportation data. For example, date/time, name of transportation company or person, or weather conditions.

New dataset 34C is generated from data 34A, 38A, 38B and the continuous sensor data.

The process is repeated for the next processing stage, storage 36C. Storage of the fodder may be in warehouses, bunkers, silos, open air, tarping and/or wrapping. Both transportation 36B and storage 36C can be provided at the same time (e.g. where the fodder is stored in a vehicle),or may be separate processes. Transportation 36B may occur several times, in sequence or independently, (e.g. several transportation steps) before storage 36C. Similarly, storage data 38C is transmitted to the monitoring device 2 via a pairing process 40C. The storage data 38C may comprise any of the following:
- Storage type. For example, the type of building or container, or whether the fodder is exposed, fully enclosed or partially enclosed.
- Storage time. This may comprise the start and/or end time of storage, or an indication of the time span between the start and end time.
- Weather conditions during storage.
- Physical conditions of the fodder. For example, temperature, moisture level, or pH.
- Localization of storage.
- Identifier of fodder.

New dataset 34D is generated from data 34A, 38A-C and the continuous sensor data.

The process is repeated for the next processing stage, usage 36D. Usage 36D may comprises feeding to cattle, use as bedding, burning and/or disposal. The result of said usage 36D is the consumption of the fodder. Similarly, usage data 38D is transmitted to the monitoring device 2 via a pairing process 40D. The usage data 38D may comprise any of the following:
- Usage type.
- Amount of fodder. The amount may comprise a remaining amount and/or a used amount.
- Fodder nutritional composition, as discussed above.
- Feed ration. For example, the amount of fodder provided in a particular feeding session or over a number of sessions.
- Weather conditions during storage.
- Physical conditions of the fodder. For example, temperature, moisture level, or pH.
- Localization of storage.

New dataset 34E is generated from data 34A, 38A-D and the continuous sensor data. The dataset 34E includes all the data for the respective processing stages 36A-36D and the continuously measured sensor data. Any of the datasets 34A-E may be extracted or transmitted as required. For example, the data 34E may be extracted by a user's device to allow viewing of the data, or may be transmitted to a cloud storage network.

Timestamps are provided for each data input (e.g. for each processing stage and continuous monitoring). Identifiers may be assigned to data during each processing stage (e.g. to identify each particular piece of equipment providing the data). This allows traceability of the fodder from initial production all the way to usage (e.g. provide an audit). The measurements taken by the monitoring device 2 further allow estimation of the quality of the fodder, thereby allowing the user to take action to correct any deficiencies.

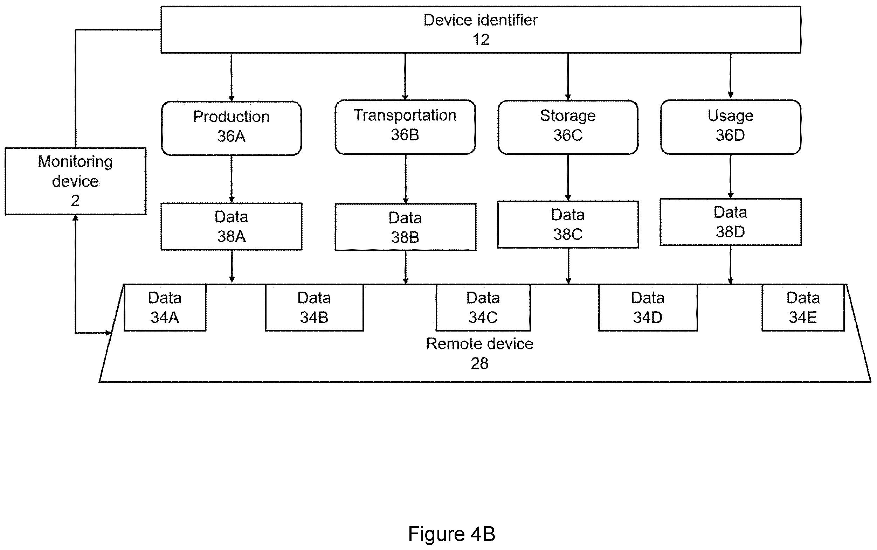

A second embodiment of the invention is shown in figure 4B. In this embodiment, the processing stage data 38A-B is transmitted to a remote device 28. The remote device 28 may comprise any suitable form, as previously discussed. The remote device 28 may comprise a remote server and/or cloud storage. The remote device 28 may comprise a plurality of different storage devices in operative communication with one another. For example, different remote devices 28 may communicate via an API or the like.

Means to detect the identifier 12 on the monitoring device 2 may be provided at each processing stage 36. For example, a camera, optical recognition device or interrogating device (e.g. RFID device) may be provided. The device at each processing stages reads, extracts or otherwise determines the identifier 12 for each unit of foodstuff. A processing stage device at each processing stage 36 may then associate the respective datasets 38 with the identifier 12 on the monitoring device 2. Timestamps and/or identifiers of processing apparatus may be associated with the data 38. The data 38 and identifier 12 may be transmitted to the remote device 28 (e.g. cloud storage). The respective datasets 38 may be aggregated/collated by the remote device 28. The processing stage device may comprise the interrogating device 26.

Measurement data 34A from the monitoring device 2 may likewise be transmitted to the remote device 2 (e.g. via mobile/cellular data). Both the measurement data 34A and the processing stage data 38 are therefore stored on the remote device. This allows easy access to all the collected data.

In some embodiments, both of the above embodiments are run concurrently. Thus, data is stored both in the monitoring device 2 and the remote device 28. Thus, data can be collected using two separate means to help ensure data integrity. The device at the processing stage may read the identifier on the monitoring device 2, and the data 38 and the associated identifier 12 may be transmitted to the device.

Once the fodder has been used or being prepared to be used shortly (i.e. just before feeding), the monitoring device 2 is removed. The monitoring device 2 may be removed from the fodder using an extractor, which will be described in detail later. During removal, the trigger sensor 24 provides an indication that the monitoring device 2 has been removed. This may provide a termination event. The monitoring system 14 ceases to monitor the properties of the fodder (e.g. the sensors 22 cease to make measurements). The device enters an idle status 44. The monitoring device 2 may power turn off, or enter a low power/maintenance mode. In some embodiments, the user may manually turn off the device (e.g. via a button switch). In some embodiments, the monitoring system 14 may be deactivated electronically/remotely. For example, a remote device may transmit a signal to terminate data collection.

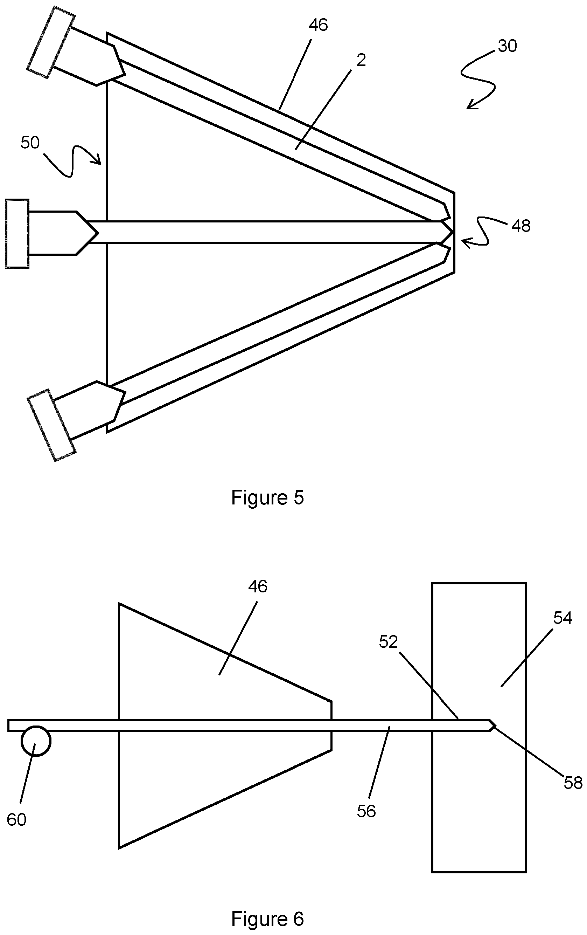

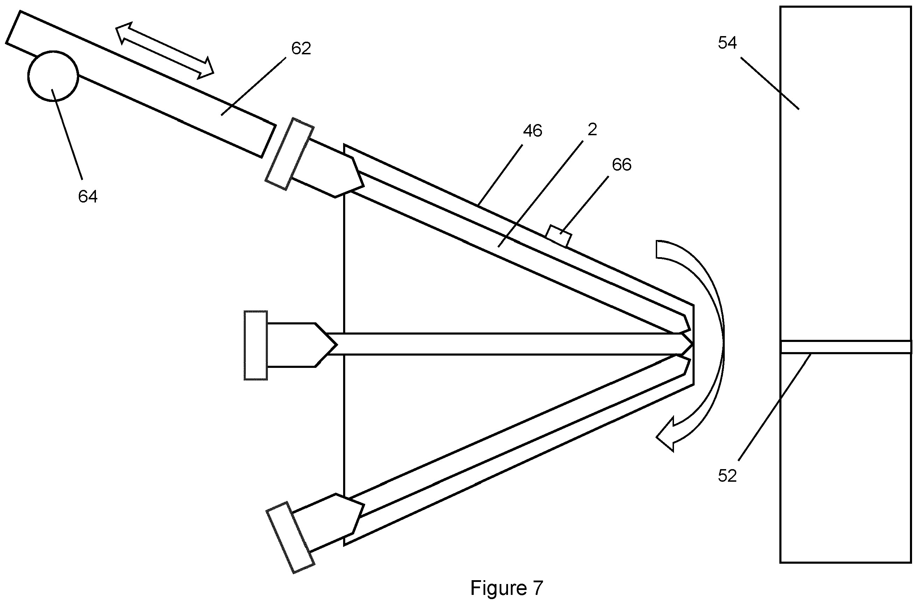

The applicator 30 is shown in further details in figures 5-7. The applicator 30 may be mounted to machinery configured to handle or process fodder. For example, the applicator 30 may be provided on any of: a forklift; a loader; a baler; or a bale wrapper. The applicator 30 may be an integral part thereof, or maybe retrofit thereon. In some embodiments, the applicator 30 may comprise a standalone device.

The applicator 30 comprises a magazine 46 configured to hold one or more monitoring device 2. The magazine 46 may comprise a conical or trapezoidal shape. The magazine may therefore taper toward a first end 48. The first end 48 of the monitoring device 2 is placed toward the first end 48 of the magazine 46. The second end 6 of the monitoring device 2 is provided at the second end 50 of the magazine 46. The tapered shape accommodates the increased width the monitoring device 2 at the second end 6. The monitoring device 2 may be held in compartments, channels, or dividers. Alternatively, the monitoring device 2 are loosely held in the magazine. Both first end 48 and the second end 50 comprises apertures to allow the device 2 to pass therethrough. The monitoring device 2 may therefore be placed into the magazine 46 and pushed out therefrom.

Referring to figure 6, the application 30 is configured to create a hole 52 or channel in the fodder 54 (the monitoring devices 2 are omitted for clarity). The hole/channel 52 allows easier insertion of the monitoring device 2. This allows the monitoring device 2 to be made of lighter/cheaper materials. The applicator 30 comprises perforator 56, such as a spike or needle or drill, configured to penetrate into the fodder 54. The spike 56 comprises a sharp/tapered tip 58. The spike 56 is retractable to allow insertion and removal from the fodder 54. An actuation mechanism 60 extends/retracts the spike 56. The actuation mechanism 60 comprises any suitable means, for example: a motor; solenoid; hydraulic actuator; electrical actuator or pneumatic actuator. Generally, the hole 52 is narrower than the width of the shaft 10 of the monitoring device 2 to ensure that the monitoring device 2 is held firmly in the fodder 54.

The spike 56 may pass through the magazine 46 and the first end 48 thereof. This creates a hole 52 in a position in which the monitoring device 2 can be pushed out. In other embodiments, the spike 56 is placed in a difference position, and the magazine is moved into alignment with the hole 52. In some embodiments, the spike 56 and actuation mechanism 60 may be provided separately from the applicator 30.

Once the hole 52 is created, then a monitoring device 2 can be pushed into said hole 52 and the fodder 54 accordingly. A pusher 62 is configured to push the monitoring device 2 out from the magazine 46. The pusher 62 is configured to engage the second end 6 of the monitoring device 2. The pusher 62 is provided at the second end 50 of the magazine 46. The pusher 62 comprises a rod or bar 64 configured to engage the monitoring device 2. The pusher 62 comprises an actuation mechanism 64 configured to drive the pusher 62. The actuation mechanism 64 comprises any suitable means, for example: a motor; solenoid; hydraulic actuator; or pneumatic actuator. The actuation mechanism 64 may comprise a rack and pinion system.

The pusher 62 pushes the monitoring device 2 out from the magazine 46 and into the hole 52. The stroke length of the pusher 62 is configured to ensure adequate insertion of the monitoring device 2.

The magazine 46 is rotatable. The magazine 46 may therefore rotate to a position where a monitoring device 2 is aligned with the pusher 62. The magazine 46 may comprise a motor or the like to provide said rotation. The magazine 46 may comprise a sensor to determine the position of the monitoring device(s) 2 within the magazine. The sensor data may be used to control the position of the magazine to synchronise the pusher 62 with rotation of the magazine to place a monitoring device 2 in front of the pusher 62. In other embodiments, the magazine may simply rotate a fixed amount after each actuation of the pusher 62 and the monitoring device 2 is assumed to be present at each actuation of the pusher 62.

Sensors may be used to confirm that the magazine 46 is aligned with the hole 52. For example, an optical or depth sensor may be used. If the magazine is not aligned with the hole 52, the applicator 30 may be moved to ensure alignment. Sensors may be used to confirm correct insertion of the monitoring device 2. For example, if the device is not properly inserted, then a notification may be provided to the user and/or the applicator 30 may be configured to stop.

In other embodiments, the magazine 46 comprise a linear shape. The magazine linearly moves to align the monitoring device 2 with the pushes, or an internal mechanism (e.g. a spring) biases the monitoring devices 2 into position whilst the magazine remains stationary. In other embodiments, the magazine comprises a cylindrical shape and comprises a plurality of exit holes for each monitoring device 2 (e.g. like a revolver pistol). In other embodiments, a feed system may provide monitoring devices 2 (e.g. the monitoring devices 2 pneumatically fed into a position in front of the pusher).

The applicator 30 may comprise a trigger 66 configured to activate the trigger sensor 24. The trigger 66 may comprise any of: a magnet; mechanical protrusion; optical emitter; or RF transmitter. Then trigger sensor 24 detects the trigger 66 and activates the monitoring device 2 accordingly. The trigger 66 may be provided part-way along the length of the magazine 46 or at/near the first end 48 thereof. This ensures the trigger sensor 24 is only triggered when the monitoring device 2 leaves the magazine. In other embodiments, the trigger 66 may be provided on the pusher 62. In some embodiments, the applicator 30 is configured to emit a wireless signal to trigger the trigger sensor 24. In some embodiments, release of the monitoring device 2 from the magazine 46 may activate the sensor. For example, the monitoring device 2 may comprise a switch or release tab that is actuated during release from the magazine or insertion into the fodder.

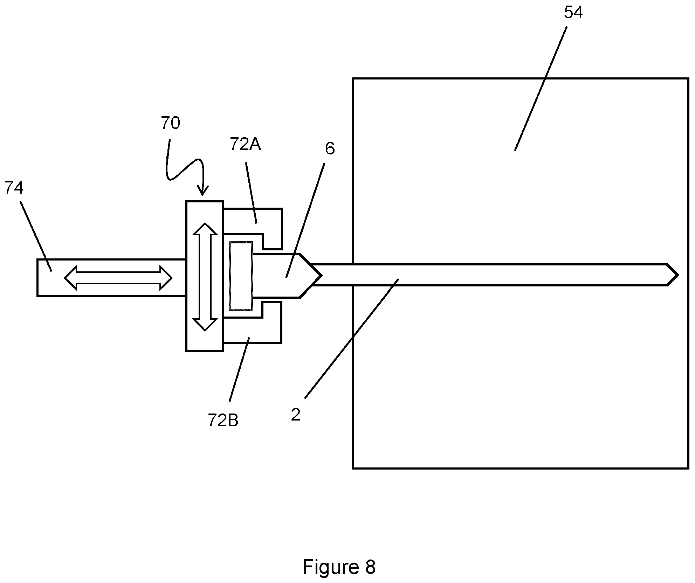

An extractor 68 is shown in figure 8. The extractor 68 comprises a jaw arrangement 70. The jaw 70 is configured to grab the second end 6 of the monitoring device 2. The jaw 70 comprises two portions 72A,B movable toward/away from one another. The jaw portions 72A,B comprise a cavity configured to receive the second end 6. The jaw portions 72A,B may comprise a C-shape sectional profile. The jaw 70 is mounted to arm or other support member 74. The arm 74 may be movable to move the jaw 70 towards/away from the fodder 54. Once the jaw 70 has gripped the monitoring device 2, then the jaw 70 is moved away from the fodder 54 to withdraw the monitoring device 2 therefrom. The trigger sensor 24 detects removal of the monitoring device 2 and monitoring stops accordingly.

The extractor 68 may be an automated device mounted on agricultural machinery, for example, a forklift, loader, dispenser used to transfer fodder. In other embodiments, the extractor comprises a standalone piece of equipment that is manually used.

In other embodiments, the monitoring device 2 may be manually removed. The device 2 may be manually turn off or put into an idle state.

The monitoring device 2 may then be re-used as required.

The present arrangement allows tracing of a foodstuff through the productions and processing process. Various properties of the foodstuff are monitored through the process, thereby allowing tracking of the quality of the foodstuff. Data is stored on a probe device, that can be conveniently retrieved, and the device is stored in the foodstuff itself. The use of an individual probe (i.e. monitoring device) and/or identifier thereon, allows tracking of individual units of foodstuff. Alternatively, the identifier on the monitoring device is used to associate processing stage data with a particular batch/unit of foodstuff, and then the data can be stored in the cloud etc. for easy retrieval. The device is configured to automatically start and stop recording data upon insertion/removal into/from the foodstuff.

## Claims

1. A method of monitoring an animal foodstuff (54) comprising:
measuring one or more property of the foodstuff using one or more sensor (22) on a monitoring device (2) inserted into the foodstuff (54) and storing said measurement data on the monitoring device (2); and
receiving by the monitoring device, (2) data (38) relating to one or more processing stage (36) of the foodstuff.

2. A method according to claim 1, comprising storing said processing stage data on the monitoring device (2) in non-volatile memory (18).

3. A method according to any preceding claim, where the property of the foodstuff (54) comprises one or more of: temperature; moisture; relative humidity; pressure; or pH of the foodstuff.

4. A method according to any preceding claim, where the processing stage (36) comprises one or more of: production; transportation; storage; or usage of the foodstuff.

5. A method according to any preceding claim, where the processing stage data (38) comprises one or more of: a geographical locating of the processing stage; weather conditions during processing; an initial nutritional content of the foodstuff; an identifier of a vehicle; an identifier of the fodder or unit/batch thereof; a storage time or type; or a feed ration of the foodstuff.

6. A method according to any preceding claim, where the monitoring device (2) comprises a sensor (24) configured to detect an initiation event, and where the monitoring device (2) initiates measuring of one or more property of the foodstuff (54) in response to said initiation event and/or where the monitoring device (2) comprises a sensor (24) configured to detect an termination event, and where the monitoring device (2) terminates measuring of one or more property of the foodstuff (54) in response to said termination event.

7. A method according to any preceding claim, where the monitoring device (2) is configured to pair with a processing stage device provided at the processing stage (36), and receive said processing stage data (38) from the a processing stage device.

8. A method according to any preceding claim, comprising receiving processing stage data (38) at each processing stage (36) and storing the data (38) for each processing stage (36) on the monitoring device (2) and concurrently continuously storing measurement data on the monitoring device (2).

9. A monitoring device (2) configured to monitor an animal foodstuff (54) comprising:
one or more sensor (22) on the monitoring device (2) configured to be inserted into the foodstuff (54) and to measure one or more property of the foodstuff (54);
a controller (16) configured to store said measurement data on the monitoring device (2); and
the controller (16) configured to receive data (38) relating to one or more processing stage (36) of the foodstuff.

10. A monitoring device according to claim 10, comprising a sensor (24) configured to detect remove and/or insertion of the monitoring device (2) into the foodstuff (54).

11. A monitoring device according to claim 10 or 11, where the monitoring device (2) comprises a first end (4) configured to be a received in the food stuff (54) and a second end (6) configured to protrude from the foodstuff (54), the second end comprising one or more identifier (12).

12. A method of monitoring an animal foodstuff (54) comprising
measuring one or more property of the foodstuff (54) using one or more sensor (22) on a monitoring device (2) inserted into the foodstuff (54), the monitoring device (2) comprising one or more identifier (12);
determining via a device at a processing stage (36) the identifier (12) on the monitoring device (2) and associating the identifier (12) with data (38) relating to the processing stage (36).

13. A monitoring system configured to monitor an animal foodstuff (54) comprising:
a monitoring device (2) comprising one or more sensor (22) configured to be inserted into the foodstuff (54) and to measure one or more property of the foodstuff (54) and comprising one or more identifier (12);
a device provided at one or more processing stage (36) of the foodstuff (54), and comprising a means to determine the identifier (12) on the monitoring device (2); and
where the processing stage device comprises a controller configured to associate the identifier (12) with data (38) relating to the processing stage (36).

14. An applicator (30) for inserting a monitoring device (2) into a foodstuff (54) comprising:
a magazine (46) configured to receive one or more monitoring device (2), the magazine (46) comprising at least one outlet (48); and
a pushing mechanism (62) configured to push at least one of the monitoring devices (2) out from the magazine (46) via the outlet (48).

15. An applicator according to claim 14, comprising a perforator (56) configured to provide a hole (52) in the foodstuff (54) such that the monitoring device (2) may be inserted thereinto.
